# EUROPÄISCHE PATENTSCHRIFT

(11) **EP 0 648 736 B1**
(45) Veröffentlichungstag und Bekanntmachung des Hinweises auf die Patenterteilung: **13.08.1997**
(21) Anmeldenummer: 94115657.2
(22) Anmeldetag: 05.10.1994
(51) Int. Cl.: C07C 209/60

(54) **Verfahren zur Herstellung sekundärer oder tertiärer Amine**
Process for the preparation of secondary or tertiary amines
Procédé de préparation d'amines secondaires ou tertiares

(30) Priorität: 13.10.1993 DE 4334809
(43) Veröffentlichungstag der Anmeldung: 19.04.1995
(73) Patentinhaber: HOECHST AKTIENGESELLSCHAFT, 65926 Frankfurt am Main (DE)
(72) Erfinder: Diekhaus, Gerhard, Dr., D-46147 Oberhausen (DE); Kampmann, Detlef, Dr., D-86368 Gersthofen (DE); Kniep, Claus, D-46049 Oberhausen (DE); Müller, Thomas, D-46535 Dinslaken (DE); Walter, Jutta, Dr., D-47506 Neukirchen-Vluyn (DE); Weber, Jürgen, Dr., D-46147 Oberhausen (DE)

(56) Entgegenhaltungen:
- GB-A- 2 113 210

## Beschreibung

Die Erfindung betrifft ein Verfahren zur Herstellung von sekundären oder tertiären Aminen durch katalytische Umsetzung höherer endständiger Olefine mit Kohlenmonoxid, Wasserstoff und einem primären oder sekundären Amin. Die Reaktion erfolgt unter Druck und bei erhöhter Temperatur in Gegenwart eines Gemisches aus Rhodium- und Rutheniumverbindungen als Katalysator.

Die Herstellung sekundärer oder tertiärer Amine aus Olefinen, Kohlenmonoxid, Wasserstoff und primären oder sekundären Aminen, diese Reaktion wird als Aminomethylierung bezeichnet, ist bekannt. Die Umsetzung erfolgt in Gegenwart von Katalysatoren, unter denen Eisenpentacarbonyl sowie Verbindungen des Rhodiums, Rutheniums oder Iridiums die größte Bedeutung besitzen. Statt Wasserstoff kann auch Wasser als Reaktant eingesetzt werden (vgl. z.B. F. Jachimowicz, J.W. Raksis, J. Org. Chem. 1982, 47, Seiten 445 bis 447). Nach einem in der GB-A-2 113 310 beschriebenen Verfahren erhält man tertiäre Amine durch Reaktion langkettiger Olefine mit Kohlenmonoxid, Wasserstoff und einem primären oder sekundären Amin in Gegenwart einer Rhodium- oder Rutheniumverbindung als Katalysator, die in einem Mono-, Di- oder Trialkohol oder einem Gemisch eines dieser Alkohole mit Wasser gelöst ist. Nach Beendigung der Reaktion trennt man Reaktionsprodukt und Lösungsmittelphase voneinander und führt das den Katalysator enthaltende Lösungsmittel in die Reaktionszone zurück. Mit dem Wiedereinsatz des Katalysators ist eine Reduzierung der Aminausbeute und die vermehrte Bildung von Nebenprodukten verbunden. Daher ist der Katalysator nur begrenzt einsatzbereit und muß zur Wiedergewinnung, insbesondere des wertvollen Rhodiums, aufgearbeitet oder regeneriert werden.

Die sekundären und tertiären Amine haben Bedeutung als Vorstufen von Verbindungen, die als Tenside eingesetzt werden. Für dieses Anwendungsgebiet ist es von Interesse, die Umsetzung der endständigen Olefine mit Kohlenmonoxid, Wasserstoff und primärem oder sekundärem Amin hinsichtlich der Bildung von tertiärem n-Amin und iso-Amin zu steuern. Das Verhältnis der beiden Isomeren im Reaktionsgemisch bestimmt nämlich die Eigenschaften der aus ihnen hergestellten Tenside.

Es bestand daher die Aufgabe für die Aminomethylierung von höheren, endständigen Olefinen einen hochaktiven Katalysator zu entwickeln, der in so geringer Menge wirksam ist, daß seine Rückführung nicht erforderlich ist und gegebenenfalls sogar auf seine Rückgewinnung verzichtet werden kann.

Überdies soll es der Katalysator erlauben, das Verhältnis von n- und iso-Verbindung im Reaktionsprodukt beliebig zu variieren.

Die Erfindung besteht in einem Verfahren zur Herstellung sekundärer oder tertiärer Amine aus endständigen Olefinen mit mindestens 6 Kohlenstoffatomen im Molekül, Kohlenmonoxid, Wasserstoff und einem primären oder sekundären Amin bei erhöhter Temperatur und erhöhtem Druck in Gegenwart eines im Reaktionsgemisch homogen gelösten Gemisches aus Rhodium- und Rutheniumverbindungen als Katalysator. Es ist dadurch gekennzeichnet, daß Rhodium und Ruthenium im Molverhältnis 1 : 2 bis 1 : 50 eingesetzt werden und die Rhodiumkonzentration, bezogen auf das Olefin, 2 bis 15 Gew.-ppm beträgt.

Überraschenderweise ist das Gemisch aus Rhodium- und Rutheniumverbindung ein überaus aktiver Aminomethylierungskatalysator, der, bezogen auf das eingesetzte Olefin, bereits bei geringer Rhodium-Konzentration wirksam ist, so daß er nicht zurückgeführt oder zurückgewonnen werden muß.

Zur Erzielung von Ausbeuten über 85 %, insbesondere über 90 %, bezogen auf das eingesetzte Olefin, ist eine Rhodiumkonzentration (bezogen auf das Olefin) von 2 bis 15 Gew.-ppm einzuhalten. Höhere oder geringere Konzentrationen mindern die Ausbeute. Bevorzugt werden auf Olefin bezogene Rhodiumgehalte von 6 bis 12 und insbesondere 8 bis 10 Gew.-ppm.

Durch die Kombination von Rhodium mit Ruthenium wird die katalytische Aktivität des Rhodiums verstärkt. Darüber hinaus kann durch Abstimmung des molaren Verhältnisses von Rhodium und Ruthenium das Verhältnis von n- und iso-Verbindung im Reaktionsprodukt beeinflußt werden. Je mol Rhodium setzt man erfindungsgemäß 2 bis 50 mol Ruthenium ein, bevorzugt werden 3 bis 30 und insbesondere 4 bis 15 mol Ruthenium je mol Rhodium.

Das im Einzelfall zu wählende molare Verhältnis der Metalle im Katalysator, um ein bestimmtes Verhältnis von n-Amin und iso-Amin im Reaktionsprodukt zu erzielen, hängt von der Rhodiumkonzentration ab. Konzentrationen an der unteren und an der oberen Grenze des für das erfindungsgemäße Verfahren charakteristischen Bereichs von 2 bis 15 Gew.-ppm Rhodium ergeben bei molaren Verhältnissen von Rhodium zu Ruthenium von etwa 1 : 3 bis etwa 1 : 6 Amingemische, die überwiegend das n-Isomere enthalten. Bei Katalysatoren, die reicher an Ruthenium sind und etwa 10 bis 20 mol Ruthenium je mol Rhodium enthalten, werden etwa äquimolare Gemische aus n- und iso-Verbindung gebildet.

Nach dem erfindungsgemäßen Verfahren werden Olefine mit mehr als 6 Kohlenstoffatomen,im Molekül, insbesondere mit 6 bis 24 und vorzugsweise 8 bis 20 Kohlenstoffatomen umgesetzt. Die Olefine können geradkettig oder ein- bzw. mehrfach verzweigt sein. Ihre Doppelbindung befindet sich an einem endständigen Kohlenstoffatom. Beispiele für geeignete Olefine sind n-Hexen-1, n-Hepten-1, n-Octen-1, n-Nonen-1, n-Decen-1, n-Dodecen-1, n-Tetradecen-1, n-Hexadecen-1, insbesondere n-Decen-1, n-Dodecen-1 und n-Tetradecen-1.

Die als Reaktionspartner der Olefine verwendeten primären oder sekundären Amine entsprechen der allgemeinen Formel R¹R²NH, wobei R¹ und R² gleich oder verschieden sind und Wasserstoff oder Alkylreste mit 1 bis 4 Kohlenstoffatomen bedeuten mit der Maßgabe, daß die Reste R¹ und R² nicht gleichzeitig H sind. Beispiele für geeignete primäre Amine, die in der handelsüblichen Form eingesetzt werden können, sind Methylamin, Ethylamin und Propylamin, insbesondere Methylamin und Ethylamin und für sekundäre Amine Dimethylamin, Diethylamin und Methylethylamin. Je mol Olefin verwendet man 1 bis 3, insbesondere 1 bis 2 und bevorzugt 1 bis 1,5 mol primäres oder sekundäres Amin.

Die Zusammensetzung des Synthesegases, d.h. das Verhältnis von Kohlenmonoxid zu Wasserstoff, kann in weiten Grenzen variiert werden. Im allgemeinen enthält das Gasgemisch Kohlenmonoxid und Wasserstoff im Volumenverhältnis nis von etwa 1 : 1 etwa 1 : 2,5, jedoch sind auch Gemische anderer Zusammensetzung geeignet. Je mol Olefin verwendet man 1 bis 12, insbesondere 1,2 bis 10, bevorzugt 1,5 bis 8 mol Kohlenmonoxid und 2 bis 24, insbesondere 2,4 bis 20 und bevorzugt 3 bis 16 mol Wasserstoff. Es hat sich bewährt, mit einem Überschuß von 10 bis 200, insbesondere 25 bis 120 mol Kohlenmonoxid und 20 bis 400, insbesondere 50 bis 200 mol Wasserstoff je mol Olefin zu arbeiten.

Besonders zweckmäßig ist es, die Reaktionspartner Olefin und Amin in Abwesenheit von Lösungsmitteln oder sonstigen Reaktionsmedien mit dem Kohlenmonoxid-/Wasserstoffgemisch umzusetzen. Das Amin kann bei dieser Reaktionsführung durch einfache Trennung von wäßriger und organischer (Produkt-)Phase gewonnen werden. Wenn es die individuellen Umstände erfordern, ist es aber auch möglich, die Umsetzung der Reaktanten in Gegenwart eines Lösungsmittels vorzunehmen. Geeignet sind Alkohole, bevorzugt Monoalkohole mit 1 bis 3 und Dialkohole mit 3 bis 6 stoffatomen im Molekül.

Katalytisch wirksam sind Rhodium und Ruthenium in Form der Carbonylverbindungen, die sich im Reaktionsgemisch unter den Reaktionsbedingungen bilden. Als Katalysatorvorstufen setzt man die Metalle in feinverteiltem Zustand oder als im Reaktionssystem lösliche oder unlösliche Verbindungen ein, z.B. Salze anorganischer oder organischer Säuren wie die Nitrate, die Formiate, Acetate oder 2-Ethylhexanoate oder auch die Oxide.

Statt den Katalysator im Reaktionsgemisch herzustellen, kann man ihn aus den Metallen oder den oben beispielhaft aufgeführten Verbindungen in einem Hilfsmedium präformieren und als Lösung dem Reaktionsgemisch zusetzen.

Die Umsetzung der Reaktanten erfolgt bei Temperaturen von 100 bis 160°, insbesondere 110 bis 150° und bevorzugt 120 bis 140°C. Der Druck beträgt 4 bis 20, insbesondere 8 bis 18 und bevorzugt 10 bis 16 MPa. Die Umsetzung wird in üblichen Druckreaktoren, die mit einer Rühr- oder Mischeinrichtung versehen sind, z.B. Rührautoklaven, durchgeführt. Man legt den Katalysator in Form seiner Komponenten suspendiert oder gelöst zusammen mit Olefin, Amin und Lösungsmittel vor und leitet in das Gemisch Kohlenmonoxid und Wasserstoff ein. Die Verweilzeit der Reaktionspartner in der Reaktionszone beträgt 2 bis 6, insbesondere 3 bis 5 h.

Die Umsetzung kann sowohl absatzweise als auch kontinuierlich durchgeführt werden.

Nach Beendigung der Umsetzung läßt man das Reaktionsgemisch erforderlichenfalls abkühlen und trennt es destillativ auf.

In den folgenden Beispielen wird das erfindungsgemäße Verfahren näher erläutert, jedoch nicht auf die beschriebenen Ausführungsformen beschränkt.

### Beispiel 1 (Vergleich)

In einem 1-l-Hubrührautoklaven werden 168 g 1-Dodecen (1 mol), 200 g Isopropanol als Lösungsmittel und 50 Gew.-ppm Ruthenium (in Form von Rutheniumacetylacetonat) vorgelegt. Mit Hilfe von Synthesegas (CO/H₂ = 1 : 1) führt man bis zum Erreichen eines Druckes von 7,5 MPa dem Reaktionsgemisch 60 g Dimethylamin (1,33 mol) zu und stellt schließlich eine Temperatur von 150°C und mit Synthesegas (CO/H₂ = 1 : 1) einen Reaktionsdruck von 15 MPa ein. Nach 5 Stunden wird keine Gasaufnahme mehr beobachtet. Man läßt abkühlen, entspannt, entnimmt das Rohprodukt quantitativ und untersucht es gaschromatographisch. Das Ergebnis ist der folgenden Tabelle zu entnehmen.

### Beispiel 2 (Vergleich)

Es wird wie in Beispiel 1 gearbeitet mit der Ausnahme, daß anstelle von Ruthenium Rhodium in Form von Rhodium-2-ethylhexanoat als Katalysator eingesetzt wird. Die Katalysatorkonzentration beträgt 3 Gew.-ppm Rhodium, bezogen auf eingesetztes Olefin. Die Ergebnisse sind der Tabelle zu entnehmen.

### Beispiel 3 (Vergleich)

Es wird wie in Beispiel 1 gearbeitet, jedoch die doppelte Rutheniummenge, also 100 Gew.ppm, bezogen auf eingesetztes Olefin, verwendet. Die Ergebnisse sind der Tabelle zu entnehmen.

### Beispiel 4 (Vergleich)

Es wird wie in Beispiel 1 gearbeitet, als Katalysator jedoch Rhodium in Form von Rhodium-2-ethylhexanoat in einer Konzentration von 6 Gew.-ppm Rhodium, bezogen auf Olefin, eingesetzt. Die Ergebnisse sind der Tabelle zu entnehmen.

### Beispiel 5

Es wird wie in Beispiel 1 gearbeitet, jedoch unter Verwendung eines Mischkatalysators aus 50 Gew.ppm Ruthenium (in Form von Rutheniumacetylacetonat) und 3 Gew.-ppm Rhodium (in Form von Rhodium-2-ethylhexanoat). Die Ergebnisse sind der Tabelle zu entnehmen.

### Beispiel 6

Es wird wie in Beispiel 1 gearbeitet, jedoch unter Verwendung eines Mischkatalysators aus 100 Gew.-ppm Ruthenium (in Form Rutheniumacetylacetonat) und 10 Gew.-ppm Rhodium (in Form von Rhodium-2-ethylhexanoat). Die Ergebnisse sind der Tabelle zu entnehmen.

### Beispiel 7

Es wird wie in Beispiel 1 gearbeitet, jedoch unter Verwendung eines Mischkatalysators aus 58,6 Gew.-ppm Ruthenium (als Rutheniumacetylacetonat) und 11,7 Gew.-ppm Rhodium (als Rhodium-2-ethylhexanoat), jeweils bezogen auf Olefin. Die Ergebnisse sind der Tabelle zu entnehmen.

### Beispiel 8

Es wird wie in Beispiel 1 gearbeitet, jedoch unter Verwendung eines Mischkatalysators aus 5,9 Gew.-ppm Rhodium (als Rhodium-2-ethylhexanoat) und 58,6 Gew.-ppm Ruthenium (als Rutheniumacetylacetonat), jeweils bezogen auf Olefin. Die Ergebnisse sind der Tabelle zu entnehmen.

### Beispiel 9 (Vergleich)

Es wird wie in Beispiel 1 gearbeitet, jedoch unter Verwendung eines Mischkatalysators aus 100 Gew.-ppm Ruthenium (als Rutheniumacetylacetonat) und 1,7 Gew.-ppm Rhodium (als Rhodium-2-ethylhexanoat), jeweils bezogen auf Olefin. Die Ergebnisse sind der Tabelle zu entnehmen.

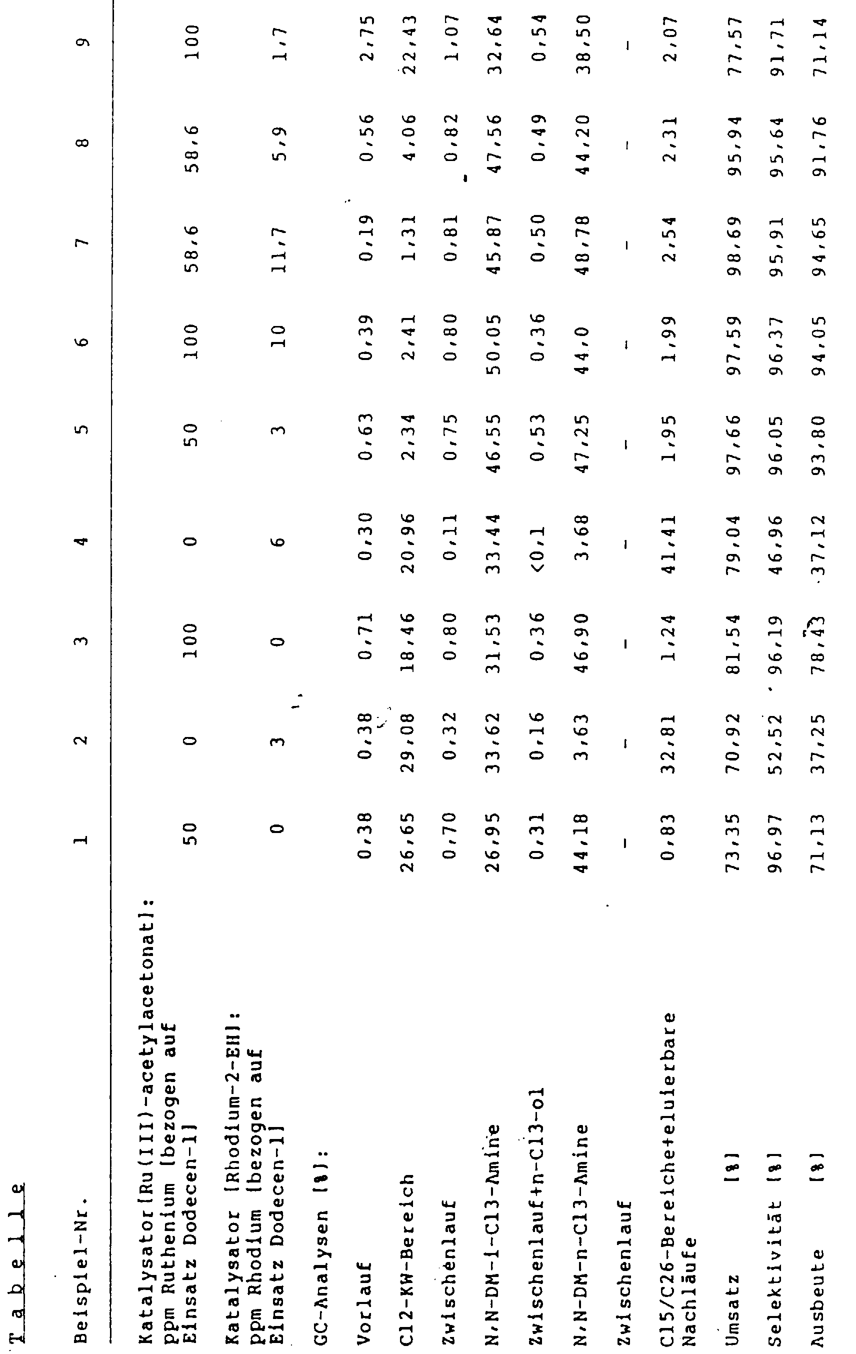

## Patentansprüche

1. Verfahren zur Herstellung sekundärer oder tertiärer Amine aus endständigen Olefinen mit mindestens 6 Kohlenstoffatomen im Molekül, Kohlenmonoxid, Wasserstoff und einem primären oder sekundären Amin bei erhöhter Temperatur und erhöhtem Druck in Gegenwart eines im Reaktionsgemisch homogen gelösten Gemisches aus Rhodium- und Rutheniumverbindungen als Katalysator, dadurch gekennzeichnet, daß Rhodium und Ruthenium im Molverhältnis 1 : 2 bis 1 : 50 eingesetzt werden und die Rhodiumkonzentration, bezogen auf das Olefin, 2 bis 15 Gew.-ppm beträgt.

2. Verfahren nach Anspruch 1, dadurch gekennzeichnet, daß die Rhodiumkonzentration, bezogen auf das Olefin, 6 bis 12, insbesondere 8 bis 10 Gew.-ppm beträgt.

3. Verfahren nach Anspruch 1 oder 2, dadurch gekennzeichnet, daß je mol Rhodium 3 bis 30, insbesondere 4 bis 15 mol Ruthenium eingesetzt werden.

4. Verfahren nach einem oder mehreren der Ansprüche 1 bis 3, dadurch gekennzeichnet, daß die Umsetzung in Gegenwart eines Lösungsmittels erfolgt.

5. Verfahren nach Anspruch 4, dadurch gekennzeichnet, daß als Lösungsmittel Monoalkohole mit 1 bis 3 und Dialkohole mit 3 bis 6 Kohlenstoffatomen im Molekül verwendet werden.

6. Verfahren nach einem oder mehreren der Ansprüche 1 bis 5, dadurch gekennzeichnet, daß die Umsetzung bei 100 bis 160°, insbesondere 110 bis 150° und bevorzugt 120 bis 140°C und bei Drücken von 4 bis 20, insbesondere 8 bis 18 und bevorzugt 10 bis 16 MPa erfolgt.

7. Verfahren nach einem oder mehreren der Ansprüche 1 bis 6, dadurch gekennzeichnet, daß die Olefine geradkettig oder einfach oder mehrfach verzweigt sind und 6 bis 24, vorzugsweise 8 bis 20 Kohlenstoffatome im Molekül enthalten.

8. Verfahren nach einem oder mehreren der Ansprüche 1 bis 7, dadurch gekennzeichnet, daß die Amine der allgmeinen Formel R¹R²NH entsprechen, wobei R¹ und R² gleich oder verschieden sind und Wasserstoff oder Alkylreste mit 1 bis 4 Kohlenstoffatomen bedeuten mit der Maßgabe, daß R¹ und R² nicht gleichzeitig Wasserstoff sind.

9. Verfahren nach einem oder mehreren der Ansprüche 1 bis 8, dadurch gekennzeichnet, daß je mol Olefin 1 bis 3, insbesondere 1 bis 2, vorzugsweise 1 bis 1,5 mol primäres oder sekundäres Amin eingesetzt werden.

## Revendications

1. Procédé de préparation d'amines secondaires ou tertiaires à partir d'oléfines à double liaison terminale avec au moins 6 atomes de carbone dans la molécule, du monoxyde de carbone, de l'hydrogène et une amine primaire ou secondaire à température élevée et haute pression en présence d'un mélange dissous de manière homogène dans le mélange réactionnel de composés de rhodium et de ruthénium comme catalyseur, caractérisé en ce qu'on utilise le rhodium et le ruthénium en rapport molaire 1:2 à 1:50 et que la concentration en rhodium, rapportée à l'oléfine, est 2 à 15 ppm en poids.

2. Procédé selon la revendication 1, caractérisé en ce que la concentration en rhodium, rapportée à l'oléfine, est de 6 à 12, notamment 8 à 10 ppm en poids.

3. Procédé selon la revendication 1 ou 2, caractérisé en ce qu'on utilise par mole de rhodium 3 à 30, notamment 4 à 15 mol de ruthénium.

4. Procédé selon l'une ou plusieurs des revendications 1 à 3, caractérisé en ce que la réaction a lieu en présence d'un solvant.

5. Procédé selon la revendication 4, caractérisé en ce qu'on utilise comme solvant des monoalcools de 1 à 3 et des dialcools de 3 à 6 atomes de carbone dans la molécule.

6. Procédé selon l'une ou plusieurs des revendications 1 à 5, caractérisé en ce que la réaction a lieu entre 100 et 160°, notamment entre 110 et 150°, et de préférence entre 120 à 140°C et à des pressions de 4 à 20, notamment 8 à 18 et de préférence 10 à 16 MPa.

7. Procédé selon l'une ou plusieurs des revendications 1 à 6, caractérisé en ce que les oléfines sont à chaîne linéaire ou ramifiée une ou plusieurs fois et qu'elles contiennent 6 à 24, de préférence 8 à 20 atomes de carbone dans la molécule.

8. Procédé selon l'une ou plusieurs des revendications 1 à 7, caractérisé en ce que les amines ont la formule générale R¹R²NH, où R¹ et R² sont identiques ou différents et représentent un hydrogène ou des restes alkyles de 1 à 4 atomes de carbone à la condition, que R¹ et R² ne représentent pas l'hydrogène simultanément.

9. Procédé selon l'une ou plusieurs des revendications 1 à 8, caractérisé en ce qu'on utilise par mole d'oléfine 1 à 3, notamment 1 à 2, de préférence 1 à 1,5 mol d'amine primaire ou secondaire.

## Claims

1. A process for the preparation of secondary or tertiary amine from a terminal olefin having at least 6 carbon atoms in the molecule, carbon monoxide, hydrogen and primary or secondary amines at elevated temperature under increased pressure in the presence of a mixture of rhodium compounds and ruthenium compounds, dissolved homogeneously in the reaction mixture, as the catalyst, which comprises employing rhodium and ruthenium in a molar ratio of 1:2 to 1:50, the rhodium concentration being 2 to 15 ppm by weight based on the olefin.

2. A process as claimed in claim 1, wherein the rhodium concentration, based on the olefin, is 6 to 12, in particular 8 to 10 ppm by weight.

3. A process as claimed in claim 1 or 2, wherein 3 to 10, in particular 4 to 15 mol of ruthenium are employed per mole of rhodium.

4. A process as claimed in one or more of claims 1 to 3, wherein the reaction is carried out in the presence of a solvent.

5. A process as claimed in claim 4, wherein a monoalcohol having 1 to 3 or dialcohol having 3 to 6 carbon atoms in the molecule is used as the solvent.

6. A process as claimed in one or more of claims 1 to 5, wherein the reaction is carried out at 100 to 160°, in particular 110 to 150° and preferably 120 to 140°C, under pressures of 4 to 20, in particular 8 to 18 and preferably 10 to 16 MPa.

7. A process as claimed in one or more of claims 1 to 6, wherein the olefin is straight-chain or mono- or polybranched and contains 6 to 24, preferably 8 to 20 carbon atoms in the molecule.

8. A process as claimed in one or more of claims 1 to 7, wherein the amine corresponds to the formula R¹R²NH, in which R¹ and R² are identical or different and are hydrogen or alkyl radicals having 1 to 4 carbon atoms, with the proviso that R¹ and R² are not simultaneously hydrogen.

9. A process as claimed in one or more of claims 1 to 8, wherein 1 to 3, in particular 1 to 2, preferably 1 to 1.5 mol of primary or secondary amine are employed per mole of olefin.
